(19) Europäisches Patentamt
European Patent Office
Office européen des brevets

(11) Numéro de publication: **0 217 816 B1**

(12) # FASCICULE DE BREVET EUROPEEN

(45) Date de publication de fascicule du brevet: **27.05.92**

(51) Int. Cl.5: **A61K 37/12**, A61K 37/04, A61K 35/16, A61L 27/00

(21) Numéro de dépôt: **86901423.3**

(22) Date de dépôt: **03.03.86**

(86) Numéro de dépôt internationale : **PCT/FR86/00068**

(87) Numéro de publication internationale : **WO 86/05097 (12.09.86 86/20)**

Le dossier contient des informations techniques présentées postérieurement au dépôt de la demande et ne figurant pas dans le présent fascicule.

(54) **PRODUIT A BASE D'ELASTINE, PROCEDE POUR SA PREPARATION ET SES APPLICATIONS BIOLOGIOUES, EN PARTICULIER EN TANT OUE BIOMATERIAUX ET SUPPORTS ARTIFICIELS.**

(30) Priorité: **01.03.85 FR 8503057**

(43) Date de publication de la demande: **15.04.87 Bulletin 87/16**

(45) Mention de la délivrance du brevet: **27.05.92 Bulletin 92/22**

(84) Etats contractants désignés: **AT BE CH DE FR GB IT LI LU NL SE**

(56) Documents cités:
**FR-A- 2 392 674**

**CHEMICAL ABSTRACTS, vol. 90, no. 11, 12 March 1979, Columbus, OH (US); R.L.BURLESON, p. 384, no. 84529**

**JOURNAL SURG. RES., 1978, vol. 25, no. 6**

(73) Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **RABAUD, Michel**
**107, rue Lamartine**
**F-33400 Talence(FR)**
Inventeur: **LEFEBURE CLEMENT, Françoise**
**6bis, avenue Edouard Manet**
**F-33160 Saint Médard en Jalles(FR)**
Inventeur: **BRICAUD, Henri**
**Chateau Roubric**
**F-33880 Cambes(FR)**
Inventeur: **SCHMIDTHAEUSLER, Roland**
**36, quai de l'Ill**
**F-67400 Illkirch-Graffenstaden(FR)**

**EP 0 217 816 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

SURGERY, 1972, vol. 72, no. 2; pp. 315-322

ANN. SURG., 1976, vol. 184, no. 5; pp. 594-600

(74) Mandataire: **Peaucelle, Chantal et al**
**Cabinet Armengaud Aine 3, avenue Bugeaud**
**F-75116 Paris(FR)**

## Description

L'invention a pour objet un produit à base d'élastine doté, notamment, de propriétés d'élasticité et de robustesse.

Elle vise également un procédé pour sa préparation ainsi que les applications biologiques de ce produit, notamment, pour l'élaboration de biomatériaux et de matériaux supports utilisables en particulier comme tissus artificiels ou supports de cultures cellulaires.

On sait que l'élastine est un constituant des éléments élastiques de l'organisme, notamment, dans le poumon, la peau, les parois artérielles, les ligaments et les cordons ombilicaux.

Il s'agit d'une protéine du type scléroprotéines résistant d'une manière générale à l'action des acides, des alcalis et des enzymes protéolytiques, à l'exception de l'élastase, pour laquelle elle constitue un substrat spécifique et qui la dégrade.

In vivo, l'élastine est protégée de l'action de l'élastase, notamment par des complexes élastase-inhibiteurs sériques. Cependant, dans divers processus pathologiques d'origine inflammatoire ou liés à l'âge, tels que l'emphysème pulmonaire ou l'athérosclérose, on observe une dégradation de l'élastine, vraisemblablement sous l'effet de l'élastase, dès un stade précoce de la maladie.

Des études menées sur le problème de l'adhérence de la fibrine à des tissus biologiques contenant de l'élastine et/ou du collagène, ont conduit à la conclusion que la fibrine se lie préférentiellement au collagène (Journal of Surgical Research, 25, 523-529 (1978)).

Cependant, aucun produit utilisable en tant que biomatériau, notamment en tant que peau artificielle, ne pouvait être déduit de cette étude et n'a d'ailleurs été proposé par les auteurs de cet article.

Au cours de travaux antérieurs, certains des co-inventeurs de la présente invention ont mis en évidence, in vitro, les complexes élastase-inhibiteurs sériques formés in vivo dans le plasma ou le sérum et ont étudié leur capacité à se fixer sur l'élastine et leur activité élastinolytique induite par une élévation du pH de 6 à 8,6.

Lors de l'étude d'une éventuelle activité élas tinolytique dans le plasma ou le sérum humain, il avait été rapporté que l'adsorption de plasma ou de sérum sur l'élastine, dans des conditions d'incubation à 37 ° C, dans un tampon acétate 0,2 M, 1 mM en acétates de calcium, de magnésium et de manganèse, de pH 6, s'accompagne d'une modification visible de l'aspect de cette protéine qui perd son caractère granuleux pour devenir floconneuse.

Une recherche explicative de ce phénomène a amené les inventeurs a constater qu'en opérant dans certaines conditions, il est possible de former et d'isoler un nouveau produit à base d'élastine doté à la fois de remarquables propriétés de solidité et d'élasticité. Un autre aspect particulièrement avantageux réside dans sa plasticité.

L'invention a donc pour but de fournir un produit nouveau à base d'élastine, dont les propriétés, en particulier de souplesse et de robustesse, permettent d'établir un biomatériau ou un matériau support sous diverses formes, en particulier sous forme de membranes, de tubulures ou de filaments, présentant des qualités satisfaisantes au regard de diverses applications biologiques.

Elle a également pour but de fournir un procédé de préparation de ce produit permettant d'obtenir les propriétés requises dans des conditions de mise en oeuvre aisée.

Elle vise également la mise à profit des qualités de ce produit pour l'élaboration de biomatériaux et de matériaux supports répondant à des besoins de grande importance par exemple pour la préparation de tissus ou l'élaboration de supports pour cultures cellulaires.

Le produit selon l'invention à base d'élastine est caractérisé en ce qu'il comprend un adduit tel qu'obtenu par réaction de monomères de fibrine et d'élastine, les monomères de fibrine étant tels qu'engendrés par action de thrombine en présence de $Ca^{++}$ sur du fibrinogène.

Il est entendu que dans le cadre de la présente invention, le terme élastine recouvre les élastines natives ou matures, leurs dérivés ou encore les élastines modifiées notamment par hydrolype.

D'une manière avantageuse, un produit de ce type possède une résistance chimique et des propriétés d'élasticité et de solidité de charpente élevées. Il présente en outre une grande plasticité et souplesse ainsi que des propriétés d'étanchéité et peut être en outre moulé, ce qui lui confère un grand interêt notamment en vue d'applications biologiques.

Selon l'un de ses aspects, le produit à base d'élastine de l'invention comprend un adduit renfermant de l'élastine et de la fibrine soluble.

On rappelle que la fibrine soluble est formée de manière intermédiaire, à partir de fibrinogène, durant le processus de coagulation, selon le schéma :

$$\text{fibrinogène} \xrightarrow[Ca^{++}]{\text{thrombine}} \text{Fibrine monomères} + \text{fibrinopeptides} \rightarrow \text{fibrine soluble} \xrightarrow[Ca^{++}]{\text{Facteur XIII a}} \text{fibrine insoluble}$$

Le produit préféré comprend un adduit élaboré à partir de fibrinogène et d'élastine. Selon un autre aspect de l'invention le produit comprend un adduit élaboré à partir de plasma et d'élastine.

Selon un autre aspect de l'invention, le produit à base d'élastine comprend également de la fibronectine.

Il a été démontré que la fibronectine se lie par affinité spécifique à l'élastine et participe donc à l'élaboration de l'adduit dont les qualités plastiques se trouvent ainsi grandement améliorées. La fibronectine peut être d'origine sanguine ; elle est alors préférentiellement obtenue par cryoprécipitation selon la technique de Pool J.G et Shannon A.E. décrite dans N.Eng.J. Med., 465, 273, 1443-1447. Elle peut également être surajoutée sous une forme purifiée à partir du sang du placenta ou de fractions de cellules en culture.

Des adduits du type de ceux renfermant de la fibronectine sont également élaborés avantageusement à partir d'élastine et de cryoglobulines.

Des cryoglobulines apropriées comprennent du fibrinogène et de la fibronectine selon des rapports variables. Il s'agit par exemple de la fraction dite anti-hémophilique A, cryodesséchée, qui comprend environ 10 à 15 g/l (c'est-à-dire par litre de produit reconstitué dans un tampon) de fibrinogène et environ 2 à 4 g/l de fibronectine.

Une autre fraction est obtenue dans le processus d'extraction du facteur VIII concentré et renferme environ 9 à 20 g/l de fibrinogène et 10 à 14 g/l environ de fibronectine.

Une autre fraction encore formée d'un complexe protéique cryodesséché comprenant en particulier 50 à 90 g/l environ de fibrinogène et 8 à 15 g/l de fibronectine.

Ce complexe, appelé Transglutine (décrit notamment par Thiébaut et al. dans la Nouvelle Presse Médicale du 11 septembre 1982 11, n° 35 page 538) comprend en outre 590± 250 u/ml de facteur XIII avec 95 ± 35 g/l environ de protéines totales et 35 à 90 % environ de protéines coagulables par la thrombine.

La fibrine soluble, ou les dérivés à partir desquels elle est produite, et l'élastine sont d'origine animale ou humaine. En particulier, l'élastine provient de ligament nucal du boeuf, ou d'aorte humaine, ou encore de cordons ombilicaux.

Les adduits des produits de l'invention présentent une grande stabilité. Il est ainsi possible de les conserver plusieurs mois sur des conservateurs biologiques classiques tels que l'azoture de sodium ou l'éthylmercrylthiosalicylate de sodium.

Selon les propriétés que l'on souhaite renforcer ou conférer à ces produits, divers additifs appropriés peuvent être incorporés, dans la mesure où ces additifs n'altèrent pas l'affinité de la fibrine soluble pour l'élastine dans l'adduit formé et leurs qualités résultantes. Les produits ainsi obtenus seront également particulièrement désignés sous l'expression de patch biologique dans ce qui suit.

Des additifs avantageux comprennent des composés destinés à renforcer les qualités mécaniques, tels que du collagène, ou des dérivés de cellulose ou de l'alginate de calcium.

L'addition de composés présentant une action de vulcanisation permet d'améliorer l'élasticité. Ces composés comprennent les composés soufrés, notamment du type de l'éthylmercrylthiosalicylate de sodium mentioné ci-dessus, ou de préference la thiourée, celle-ci ne renfermant pas avantageusement du mercure. D'autre part, la stérilisation par rayons $\gamma$ induit également, outre une réticulation parallèle au réseau primitif, un processus de vulcanisation indépendant de l'addition de l'agent vulcanisant, sans altérer les qualités du produit.

En outre d'autres protéines adhésives tels que le complexe FVIII (antihémophilique A), le facteur von Willebrand, la thrombospondine, la laminine, qui proviennent du sang, de la paroi vasculaire, du placenta ou de fractions de cellules en culture peuvent être ajoutés avantageusement pour améliorer les qualités mécaniques, élastiques et adhésives du biomatériau.

Le procédé de l'invention, pour la préparation des produits à base d'élastine définis ci-dessus, est caractérisé en ce qu'il comporte une étape d'incubation de fibrinogène ou d'un milieu le renfermant, en

particulier du plasma, et d'élastine, en présence de thrombine et d'ions $Ca^{++}$ , avantageusement dans des conditions pratiquement physiologiques, en particulier en ce qui concerne la température et le pH, afin que le fibrinogène, sous l'action de la thrombine activée par les ions $Ca^{++}$, se transforme en fibrine soluble, ce qui se traduit par l'apparition d'un produit floconneux ou fibrillaire dans le mélange réactionnel.

La température, la concentration en ions $Ca^{++}$ et le pH se sont révélés des facteurs prépondérants, traduisant l'intervention d'un système enzymatique.

L'étape d'incubation est réalisée avantageusement à 37° C environ.

Les produits mis en oeuvre sont équilibrés de préférence à un pH voisin de 7,4.

La teneur en $Ca^{++}$ libre correspond avantageusement à la teneur in vivo, à savoir, de l'ordre de 2 mM.

L'élastine mise en oeuvre est d'origine animale ou humaine et provient ds organes sus-indiqués.

Dans la variante comportant la mise en oeuvre de plasma comme source de fibrinogène, il s'agit de plasma provenant de sang recueilli sur anticoagulant contenant généralement un tampon citrate, de l'acide éthylène diamine tétracétique (EDTA), ce qui entraîne une complexation des ions $Ca^{++}$, ou encore de l'héparine.

Afin de libérer ces ions $Ca^{++}$ en vue de l'activation de la thrombine pour transformer le fibrinogène comme souhaité, le plasma est soumis à un traitement préalable consistant avantageusement en une dialyse.

L'opération de dialyse est effectuée contre un tampon de caractéritiques proches de celles du milieu physiologique en particulier, dont le pH et la teneur en $Ca^{++}$ répondent aux exigences ci-dessus. L'élastine est également mise en équilibre dans ce tampon.

Parmi des tampons de ce type particulièrement appropriés, on citera un tampon phosphate de pH 7,4 de composition : phosphate 1 mM, NaCl 150 mM, $Ca^{++}$ 2 mM, Mg 1 mM.

Lors de la dialyse, l'agent complexant des ions $Ca^{++}$ est éliminé du compartiment de dialyse et le fibrinogène est transformé en fibrine soluble, puis en fibrine insoluble selon le schéma rappelé ci-dessus.

La durée de dialyse est avantageusement de l'ordre d'au moins 60 minutes.

L'étape d'incubation réalisée dans les conditions définies ci-dessus conduit immédiatement, à savoir en l'espace de quelques minutes, à la transformation physique de l'élastine qui de l'état de poudre granulaire, devient fibrillaire, présentant l'aspect d'un tissu conjonctif dilacéré.

Par agitation du mélange réactionnel, on obtient la formation de fibres qui sont séparées, par exemple par centrifugation.

Pour disposer de biomatériaux utilisables dans les applications envisagées ci-après, on opère avantageusement come suit :

- On ajoute successivement à l'élastine les additifs permettant d'améliorer en particulier les propriétés mécaniques, élastiques ainsi que la réticulation du produit recherché, puis la fibrine ou un composé, ou composition source de fibrine. Lorsqu'on utilise du fibrinogène, ou un composé le renfermant, comme source de fibrine, on ajoute de la thrombine au mélange réactionnel pour obtenir la transformation du fibrinogène en fibrine. Le mélange est ensuite incubé à une température de l'ordre de 37° C jusqu'à formation du patch biologique. Une dispersion satisfaisante des différents éléments ajoutés dans le mélange est assuré par un mélangeur de type VORTEX.

Les propriétés élastiques et mécaniques confèrent au patch biologique un caractère moulable, ce qui permet d'obtenir des surfaces membranaires de formes et dimensions souhaitées pour une application donnée.

Il est ainsi possible d'obtenir le patch biologique sous forme de tubulures, filaments, ou encore de membranes ou matériaux ayant la forme de la matrice utilisée pour les préparer.

Le patch biologique obtenu est démoulé et, le cas échéant, séché par exemple sur du papier filtre pour éliminer l'excès d'eau de la préparation, et utilisé tel quel.

Aux fins de conservation, le patch est placé dans l'alcool, en particulier de l'éthanol, et scellé dans une enveloppe étanche sous gaz inerte tel que l'azote. On soumet cette enveloppe de conservation avantageusement à un rayonnement afin de stériliser le patch, ce traitement permettant également d'améliorer la vulcanisation et la réticulation.

Lorsque le patch est utilisé comme membrane support pour des cultures cellulaires, il est préférable de le conserver et de le stériliser sans enlever au préalable l'excès d'eau de la préparation qui le renferme.

Pour une application comme biomatériau, tissu artificiel, élément de réparation tissulaire ou analogue, il est avantageux de conserver le patch dans de l'alcool. Au moment de l'utilisation, on enlève alors l'excès d'alcool, par exemple avec du papier filtre, puis on trempe le patch dans plusieurs bains de sérum physiologique.

On notera également que le patch de l'invention peut être former in situ dans les milieux utilisés pour les cultures des cellules.

L'étude des propriétés des produits à base d'élastine selon l'invention, a permis en outre de mettre en évidence leur stabilité élevée. Grâce à leurs propriétés de robustesse et d'élasticité, auxquelles s'ajoutent avantageusement des qualités de biocompatibilité et d'étanchéité, les produits à base d'élastine selon l'invention sont utilisables notamment comme biomatériaux.

L'invention vise donc également de nouveaux biomatériaux comprenant des adduits ou des patchs biologiques a base d'élastine tels que définis ci-dessus.

Parmi les patchs biologiques préférés selon l'invention, on citera notamment celui comprenant essentiellement de l'élastine, de la fibrine, de la fibronectine et du collagène ainsi que celui comprenant, en plus des quatre éléments précédents, de la thiourée.

Ces biomatériaux présentent plus spécialement un grand interêt en tant que" peaux artificielles" ou "tissus conjonctifs artificiels".

Ils constituent notamment des pièces de soutien, de renforcement ou d'obturation, utilisées en association avec des colles biocompatibles et/ou des sutures, particulièrement précieuses en chirurgie.

Parmi les colles biocompatibles, on citera notamment la Transglutine qui possède également des propriétés d'adhésivité.

Il faut préciser que les propriétés thrombogéniques de ces biomatériaux favorisent également leur utilisation comme moyen de réparation des brèches tissulaires. En effet l'interaction des composants du sang, plaquettes et protéines de la coagulation, au niveau de l'interface du sang et d'un patch biologique permet l'activation des plaquettes, leur adhésion et la sécrétion du contenu de leurs granules, entrainant ainsi la formation d'agrégats plaquettaires favorisant la fixation de l'adduit au niveau de la brèche tissulaire.

Les produits selon l'invention jouent le rôle d'un tissu de soutien temporaire qui initie d'abord puis guide le processus de réparation tissulaire. Ces propriétés permettent d'appliquer ces produits dans tous les domaines de la chirurgie : notamment digestive, vasculaire, urinaire, génitale et obstétricale, maxilofaciale et plastique, dermatologique, foetale et néonatale ainsi que vétérinaire.

Ils permettent de compenser certaines pertes de tissus et en particulier de traiter des brûlés.

Selon un autre aspect, les produits de l'invention constituent des matériaux supports paticulièrement interessants comme supports de culture cellulaire.

Des cultures de cellules musculaires lisses, de fibroblastes, de cellule épithéliales et de cellules endothéliales peuvent être effectuées avec des rendements élevés.

Ils sont également utilisables comme greffons de cultures, constituant des socles dermiques sur lesquels on ensemence par exemple des cellules épithéliales qui se multiplient, avec un grand rendement, pour former un épiderme. Ces greffons, compte tenu de leur caractère biocompatible, sont transplantables sur l'organisme du receveur.

En tant que supports, ils sont utilisables en outre pour le greffage de produits biologiques, tels que des enzymes, notamment ou des inhibiteurs spécifiques ou généraux des enzymes reponsables de la protéolyse de l'élastine , du collagène, du fibrinogène et de la fibronectine .

Des supports de ce type comprennent, par exemple, incorporés dans les produits à base d'élastine, des enzymes telles que l'$\alpha$ -2 macroglobuline, l'aprotinine, l'$\alpha$ -1- antitrypsine ou des antiplasmines.

Selon un autre aspect, l'invention vise également les kits renfermant les éléments nécessaires pour effectuer une culture cellulaire ou une réparation tissulaire.

Un type de kit préféré comprend un récipient, par exemple du type boite de Petri renfermant le patch biologique d'épaisseur variable selon la culture de cellules à effectuer.

Un autre type de kit comprend le patch, avantageusement dans son enveloppe de conservation, et un flacon contenant la colle biologique, notamment la Transglutine.

D'autres caractéristiques et avantages de l'invention sont rapportés dans les exemples qui suivent relatifs à la préparation de produits selon l'invention et à leurs applications comme "tissus artificiels".

Dans ces exemples, on se réfère aux figures 1 à 5 qui représentent respectivement :

- des photos illustrant la formation d'un adduit à base d'élastine et de fibrine (figures 1a à 1d).
- des courbes correspondant à la variation, lors de la dialyse contre un tampon phosphate, des teneurs en $Ca^{++}$, $Mg^{++}$, en fonction du temps (figure 2) et en fonction de la teneur en $Ca^{++}$ du tampon de dialyse (figure 3)'
- des courbes montrant la formation de fibrinopeptides A et la diminution de la teneur en fibrinogène en fonction du temps (figure 4), et
- le temps de formation de l'adduit en fonction de la quantité de thrombine ajoutée (figure 5).

La provenance des matériaux mis en oeuvre est précisée ci-après.

On utilise de l'élastine de ligament nucal de boeuf fournie par EUROBIO, Paris, provenant de Worthington.

L'élastine d'aorte humaine est préparée selon la méthode de LANSING décrite par LEPPERT et dans

Arch. Biochem. Biophys. 1983 ; 222 : 53-58. L'élastine est lavée intensément puis gonflée à l'aide d'un tampon approprié à 37° C, juste avant utilisation.

Les échantillons de plasma humain sont obtenus sur citrate et maintenus à -40° C.

Le fibrinogène humain ( qualité L) provient de KABI DIAGNOSTIC, Stockholm, Suède. Le radio-marquage est effectué selon la technique de GREENWOOD et al décrite dans Biochem. J. 1963 ; 39 : 114-123. On dissout 10 mg de fibrinogène dans 1 ml de PBS 0.1 M, pH 7,5. Le fibrinogène est marqué avec 500 µCi d'iode 125. Pour éviter la réduction du fibrinogène marqué par le métabisulfite, on n'effectue pas l'étape de réduction, et, immédiatement après l'oxydation de $I^-$ en $I^+$, on applique le produit brut directement et rapidement sur une colonne aux fins de gel filtration (Séphadex® G 50). On obtient 5 x $10^6$ cpm/mg de fibrinogène -$^{125}I$.

Comme thrombine humaine, on utilise le produit fourni par Ortho Diagnostic systems Inc, dosé à 50 u/ml.

L'hirudine est un produit fourni par OSI Paris, provenant de Sigma.

Les taux en $Ca^{++}$ et $Mg^{++}$ sont déterminés par absorption atomique.

Les concentrations en fibrinogène dans le plasma sont mesurées selon la méthode Von Clauss et al décrite dans Acta Hemat. 1957 ; 17 ; 237-247., et les teneurs en citrate par la méthode enzymatique de ZENDER et al (Clin. Chim. Acta 1969 ; 24 : 335-340).

On mesure le fibrinopeptide A humain en utilisant le test immuno-enzymologique en phase solide décrit par SORIA et al dans Thromb. Research 1980 ; 20 : 425-435.

Exemple 1 : Préparation d'un adduit à base d'élastine de ligament nucal de boeuf et de fibrine soluble provenant de la fraction facteur VIII de cryoglobuline.

Dans un tube à hémolyse de 5 ml, on lave soigneusement 50 mg d'élastine à l'aide d'un tampon phosphate de pH 7,4 (phosphate 1 mM, NaCl 150 mM, $Ca^{++}{}_2$ mM, Mg 1 mM). On élimine le tampon de lavage par centrifugation.

On place l'élastine dans 1,5 ml de tampon phosphate. On ajoute un conservateur (par exemple de l'azoture de sodium, à raison de 200 mg/l).

On effectue une dispersion de l'élastine à l'aide d'un Vortex, puis on ajoute 0,5 ml de facteur VIII (renfermant 15 mg de fibrinogène et 8 mg de fibronectine dans 1,5 ml de tampon phosphate contenant également en plus de l'azoture de sodium).

On effectue également une dispersion du mélange à l'aide d'un Vortex.

A la température ambiante, on ajoute 6 unités de thrombine (50 µl d'une solution de Thrombase 500). Après agitation à l'aide d'un Vortex, on coule le mélange dans un moule préalablement enduit de thrombine pour faciliter ensuite le démoulage, et on soumet à incubation à 37° C pendant environ 30 min. Le produit est récupéré, lavé et séché.

En variante, au lieu de verser le mélange dans un moule, on effectue l'incubation dans le tube de réaction et on sépare le produit formé par centrifugation.

Exemple 2 : Préparation d'un produit comprenant un adduit de fibrine et d'élastine à partir de plasma humain citraté et d'élastine d'aorte humaine.

On dialyse 10 ml de plasma humain citraté contre trois charges de trente minutes chacune, du tampon phosphate de pH 7,4 décrit plus haut.

A la fin de ces opérations, le compartiment de dialyse ne renferme plus de fibrinogène, mais ne renferme pas encore de fibrine insoluble.

On ajoute environ 1 g d'élastine, lavée, gonflée et équilibrée à l'aide du même tampon, à 37° C.

Le plasma dialysé est ajouté à l'élastine en suspension dans le tampon, à raison de 10 ml de volume final par gramme, sous faible agitation à la main.

La réaction se produit immédiatement et est arrêtée par centrifugation à 3 000 t/mn durant 2 min.

On sépare le produit formé de la fibrine insoluble (caillot), qui précipite à nouveau, et on le lave abondamment avec le tampon phosphate.

Le produit se présente sous forme quasi-fibrillaire lorsqu'on soumet la suspension à une faible agitation manuelle et forme une membrane lorsqu'on laisse la réaction s'effectuer.

L'excès de fibrine insoluble participe, comme le collagène, à la cohésion finale du biomatériau.

Sur la figure 1, on a rapporté les photos représentant respectivement :

a) la suspension d'élastine seule dans le tampon phosphate,

b) le caillot de fibrine au début de sa formation,

c) l'adduit après agitation manuelle du mélange d'élastine et de plasma, et
d) l'adduit formé lorsqu'on laisse le mélange au repos.

Exemple 3 :

1. proprietes mecaniques et etudes en microscopie à balayage d'un patch biologique à base d'elastine.

Matériel et Méthodes

1) Le nouveau tissu conjonctif artificiel ou patch est constitué de fibrogène et de fibronectine isolés du plasma humain ainsi que d'élastine humain- ou bovine, l'ensemble étant associé sous l'action de la thrombine en présence de calcium. A cette préparation standard, ont été ajoutés du collagène, de l'aprotinine, de la thiourée ou de l'héparine. Ces deux derniers constituants agissent comme agents de réticulation.
2) Une étude structurale a été réalisée en microscopie électronique à balayage pour chaque type d'échantillon de patch obtenu.
3) Les propriétés de résistance mécanique de chaque échantillon ont été également testées. Leur force de rupture et leur module d'élasticité ont été évalués afin de définir le patch le plus résistant.

Resultats

1) Caractéristiques chimiques

De l'association d'élastine, de produits dérivés du fibrogène et de fibronectine en présence de thrombine, naît une nouvelle matrice tissulaire stable dans des conditions physiologiques de pH et de concentration ionique.

2) Caractéristiques structurales

En microscopie électronique à balayage, le patch présente une trame réticulée, alors que l'élastine isolée apparaît sous la forme de fibrilles dépourvues de toute connection. Le collagène et l'aprotinine ne modifient pas la réticulation du patch mais se déposent dans les mailles de la trame. Au contraire, la thiourée et l'héparine améliorent sa réticulation. Une telle étude au microscope électronique montre qu'une partie fibrillaire du plasma apparait liée à l'élastine . La liaison apparait très forte tout le long de l'élastine avec le dérivé de fibrinogène.

3) Caractéristiques physiques

La résistance mécanique du patch déterminée à l'aide d'un extensiomètre, est de 16,7 ± 2,32 g/10 $mm^2$. L'utilisation d'élastine humaine l'augmente de 63,6 % ($p > 0,05$) et l'adjonction de thiourée de 140 % ($p < 0,001$). Le patch présente un module d'élasticité de 9,28 ± 0,69. Il s'accroit de 272 % par la présence d'élastine humaine et de thiourée.

Exemple 4 : résultats relatifs aux conditions de formation d'un produit selon l'invention :

1) ETUDE DE L'INFLUENCE DE LA TENEUR EN $Ca^{++}$.

a) On rapporte sur la figure 2 les résultats obtenus, concernant les teneurs en $Ca^{++}$, en citrate et en fibrinogène, en fonction du temps, en dialysant un plasma citraté contre un tampon phosphate.

Les quatre courbes données sur cette figure correspondent aux mesures suivantes :

- Courbe O...O... : teneur en $Ca^{++}$ en utilisant pour la dialyse un tampon phosphate 1mM, $CaCl_2$ 1 mM, $MgCl_2$ 1 mM, NaCl 150 mM, pH 7,4.
- Courbe ●...●... : teneur en $Ca^{++}$ avec un tampon de dialyse comme ci-dessus, mais avec $CaCl_2$ 2 mM.
- Courbe △...△... : teneur en citrate en $gl^{-1}$.
- Courbe □...□... : teneur en fibrinogène en $gl^{-1}$.

On constate que la teneur en $Ca^{++}$ dans le compartiment de dialyse reste inchangé lorsqu'on utilise un tampon de dialyse 1 $mMl^{-l}$ $Ca^{++}$, et ce bien que la teneur en $Ca^{++}$ du plasma humain normal soit

d'environ 2 $mMl^{-1}$.

Il apparaît, en outre, que, lorsque le même plasma est dialyse contre un tampon $PO_4$ à $2mMl^{-1}Ca^{++}$ (c'est-à-dire que la concentration en $Ca^{++}$ est la même à l'intérieur et à l'extérieur du compartiment de dialyse), la concentration en $Ca^{++}$ du plasma augmente jusqu'à environ 3 $mMl^{-1}$, après 2 heures de dialyse.

Durant ce processus, le citrate est éliminé du compartiment de dialyse et le fibrinogène transformé en fibrine soluble puis insoluble selon le schéma rappelé plus haut.

b) Sur la figure 3, on rapp orte les courbes de variation de la concentration en citrate et en fibrinogène en fonction de la concentration en $Ca^{++}$ du tampon de dialyse, après 30 min de dialyse contre un tampon $PO_4$ 1mM, NaCl 150 mM, $MgCl_2$ 1mM, $CaCl_2$ 0 ; 0,1 ; 0,5 ; 1,0 ; 1,5 et 2 mM, pH 7,4.

Les différentes mesures sont représentées par les courbes suivantes :

Variation de la concentration :

- en $Ca^{++}$ : courbe O...O...,
- en $Mg^{++}$ ($mMl^{-1}$) : courbe ●...●...,
- en citrate ($gl^{-1}$ ) : courbe △...△...,
- en fibrinogène ($gl^{-1}$) : courbe □...□....

On constate, qu'excepté pour $Mg^{++}$, la variation de concentration en citrate et en fibrinogène dépend de la concentration en $Ca^{++}$ du tampon de dialyse. La teneur en $Ca^{++}$ diminue légèrement avec une concentration nulle en $Ca^{++}$ du bain puis augmente lorsque la teneur en $Ca^{++}$ du bain croît.

2) ETUDE DE LA TRANSFORMATION DU FIBRINOGENE

Sur la figure 4, on représente les résultats obtenus concernant la teneur en fibrinogène dans le compartiment de dialyse en fonction du temps (courbe □...□...) et la teneur en fibrinopeptides A (courbe

●——●).

Il apparaît clairement que la teneur en fibrinogène décroît alors que celle en fibrinopeptides A augmente.

3) ETUDE DU MECANISME DE FORMATION DE L'ADDUIT

On ajoute 1,2 ou 3 ml de plasma dialysé sur 100 mg d'élastine et de 12 ml de fibrinogène contenant un aliquot de fibrinogène $^{125}I$ dans 10 ml (volume total) de tampon phosphate pH 7,4.

Les quantités d'adduit sont déterminées après lyophilisation .

Les résultats obtenus sont rapportés dans le tableau suivant :

| Plasma ml | 1 | 2 | 3 |
|---|---|---|---|
| Surnageant CPM $10^{-3}$ | 300 | 300 | 300 |
| Adduit CPM $10^{-3}$ | 750 | 800 | 910 |
| Adduit mg | 94 | 100 | 104 |

Ces résultats montrent que la plus grande partie de la radioactivité est retenue sur le produit formé et

qu'une faible quantité de fibrine est nécessaire pour former l'adduit.

4) INFLUENCE DE LA THROMBINE

Sur la figure 5, on rappporte les résultats obtenus en étudiant l'effet de l'addition de quantités croissantes de thrombine (u $ml^{-1}$, volume total) à une suspension de 100 mg d'élastine avec 15 mg de fibrinogène humain marqué à l'aide de $^{125}I$ dans 10 ml de tampon phosphate pH 7,4. On constate que l'adduit se forme serlon un rapport inverse avec le temps.

Lorsqu'on ajoute une quantité donnée de thrombine (0,1 u/ml) à l'élastine (100 mg), avec des quantités croissantes de fibrinogène $^{125}I$, on constate que le temps de formation de l'adduit dépend de la concentration en fibrinogène.

En outre, lorsqu'on ajoute au préalable de l'hirudine, qui est un inhibiteur spécifique de la thrombine, au système élastine-plasma, on n'observe aucun effet.

Exemple n° 5 : préparation d'un patch biologique.

On utilise 500 mg d'élastine d'aorte humaine, préparée comme indiqué ci-dessus, lavée avec un excès de tampon phosphate, pH 7,4.

On centrifuge la préparation et on récupère l'élastine.

On ajoute successivement 2 ml de tampon phosphate 7,4, 0,3 ml d'aprotinine telle que celle commercialisée sous la marque Zymofrène par Specia, 0,2 ml d'une solution de thiourée à maison de 20 mg/ml de tampon phosphate pH 7,4. On forme une suspension dispersée à l'aide d'un mélangeur du type VORTEX et on ajoute dans l'ordre, 3 ml de cryoprécipité essentiellement formé de fibrinogène (40 g/l) et de fibronectine (8 à 10 g/l) dans le tampon phosphate, 2 ml d'une solution de collagène hydrosoluble (type I), à raison de 2 mg/ml, 200 microlitres d'une solution de thrombine (c'est-à-dire 25 unités telle que celle commercialisée sous la marque Thrombase 500).

Entre chaque addition, on agite vigoureusement le mélange à l'aide du VORTEX pour obtenir une suspension très dispersée.

De manière avantageuse, on ajoute dans le moule dans lequel on coule la préparation la même quantité de thrombine à savoir 25 unités, ce qui permet de mieux décoller ultérieurement le patch biologique formé.

L'ensemble est incubé à 37° C durant environ 30 à 60 minutes.

Le patch biologique est ensuite démoulé et séché durant environ 1 heure entre du papier filtre afin d'éliminer l'excès d'eau de la préparation.Lorsque le patch est destiné à une application comme support de culture cellulaire, l'étape de séchage n'est pas effectuée.

Aux fins de conservation, on scelle le patch dans une enveloppe de conservation sous azote et on le soumet à un rayonnement $\gamma$ pendant environ 6 heures (rayonnement de 2,5 M-Rads) lorsque le produit est destiné comme élément de réparation, on effectue les étapes de scellement et de rayonnement en présence d'alcool, ce dernier étant alors éliminé au moment de l'utilisation à l'aide de papier filtre et le patch étant trempé dans plusieurs bains de sérum physiologique.

Exemple 6 : Application comme "peau artificelle" étude de l'aptitude au collage

Expérience n° 1

Principé :

On effectue un test de résistance mécanique à l'arrachement d'un carré de membrane formé d'un produit selon l'exemple 1, collé d'une part sur le dos d'une souris sacrifiée, d'autre part sur un carré de peau d'animal.

Un crochet fixé au carré de peau transmet la force exercée par une quantité croissante d'eau mesurée à la burette. L'arrachement total du carré de peau correspond à la force de traction maximale exprimée en gr. La colle utilisée est constituée par les cryoglobulines correspondant au produit appelé Transglutine dont il est question plus haut.

Mode opératoire :

On sacrifie une souris d'environ 20 g par confinement dans un récipient contenant de l'éther.

On fixe l'animal sur une planchette, le dos étant tourné vers le haut. On découpe un carré de 2 cm/2

cm qu'on redécoupe en quatre carrés de 1 cm/1 cm.

On enduit le carré de membrane de l'invention avec une colle biologique constituée par une préparation de Transglutine, à raison de 0,05 ml pour 1 $cm^2$. Le carré de membrane ainsi préparé est appliqué sur le dos préalablement imprégné de thrombine (Thrombase 500).

On enduit ce carré avec la Transglutine et on applique un carré de peau de l'animal ci-dessus.

On laisse reposer 30 minutes, puis on accroche par le centre du carré le dispositif de traction. On vide progressivement la burette, jusqu'à décollement total du carré de peau. La quantité d'eau mesurée ne s'avère pas inférieure à 60 g.

Résultats :

On effectue une série de quatre mesures sur le même animal et on prend la moyenne des valeurs obtenues après 30 minutes de collage.

Les valeurs obtenues sont comprises entre 62 et 116 g. Aucune mesure ne s'avère inférieure à 60 g.

Les résultats obtenus montrent la résistance de la membrane selon l'invention, cette membrane restant collée à la peau lors de l'arrachement.

etude de la biocomptabilité :

Les éléments collés restent bien en place et sur une observation de plusieurs semaines, on n'observe aucune intolérance.

Expérience n° 2

les propriétés adhésives de la Transglutine ont été quantifiées par deux dispositifs de traction adaptés pour mesurer des forces de 0 à 10 N.

Le collage est effectué sur de la peau de souris provenant d'un animal fraîchement sacrifié.

- dispositif n° 1 :

Le lambeau cutané interne dorsal de la souris est découpé en 4 carrés de 1 $cm^2$ de surface . Le dos de l'animal est imprégné d'une solution de thrombine à 500 U et de $CaCl_2$ 40 mM. Sur chaque carré de peau on applique 50 $\mu$l de Transglutine, puis on l'encolle immédiatement sur le dos de la souris. après un temps de prise de 40 minutes, un dispositif d'accrochage est relié au carré de peau collé et une traction continue est éxercée jusqu'au décollement de la peau. Un indicateur de force électronique donne la valeur de la force nécessaire au décollement. Sur les 70 lots étudiés de Transglutine, la valeur moyenne est de 0,98 ± 0,15 N (moyenne ± écart-type)

- dispositif n° 2 :

Les carrés de peau de souris sont collés sur la base de deux cylindres de 1,6 $cm^2$ de surface au moyen d'une colle cyanoacrylique à prise rapide. Les bases des cylindres sont ensuite pressées l'une contre l'autre pendant 40 minutes, en ayant préalablement déposé la Transglutine entre les deux lambeaux de peau. Puis une traction continue est exercée. Les valeurs obtenues sont plus élevées qu'avec le premier dispositif et l'arrachement obtenu est une rupture franche, sans composante élastique. Sur les 20 lots étudiés de Transglutine la valeur moyenne est de 3 ± 0,6 N.

Ces deux dispositifs ont également servi à mesurer la qualité du collage d'un patch biologique à base d'élastine et de fibrine. Un patch de 1 $cm^2$ est inséré entre les lambeaux de peau de souris et encollé par 50 $\mu$l de Transglutine en présence de thrombine. Sur 10 expériences, la valeur moyenne de la force de décollement est de 0,68 ± 0,1 N.

Exemple n° 7 : application en chirurgie digestive :

Mode opératoire :

Une perte de substance de 7 ou 10 mm de diamètre a été pratiquée dans la paroi caecale du rat Wistar ou du lapin néo-zélandais. Le trou ainsi obtenu a été colmaté à l'aide d'un patch d'un diamètre double du trou, collé à la paroi intestinale grâce à la colle biologique. Les animaux ont été sacrifiés tous les 5 jours

jusqu'au vingtième jour post-opératoire puis les trentième jour, quarantième et quatre vint dixième jours. Le caecum a été prélevé et analysé d'un point de vue macroscopique et microscopique.

Résultats : A l'échelle macroscopique, la cicatrisation quasi complète de la brêche intestinale a été observée entre le quinzième et le trentième jour post-opératoire chez le lapin. L'étude au microscope électronique à balayage montre l'existence d'une hypertrophie muqueuse due à la présence de replis de type villositaire. Observée au microscope photonique, cette muqueuse est constituée, à ce stade, d'un épithélium continu d'aspect normal tapissant un axe conjonctivo-vasculaire. La continuité de la couche musculaire ne se rétablit qu'à un stade utérieur. La coloration spécifique de l'élastine par l'orcéine montre que ce matériau est rejeté au niveau de la couche séreuse ou il s'entoure d'un matériel fibreux au-delà du trentième jour post-opératoire. Ce matériau a disparu complètement chez le rat au quarantième jour post-opératoire.

Ainsi le patch biologique, selon l'invention, permet la cicatrisation du colon chez le lapin et le rat. Ce biomatériau inverse la phénoménologie cicatricielle normale puisque la restauration de la couche muqueuse précède celle du tissu musculaire. Ces résultats prometteurs permettent de la proposer comme matériau de choix dans le traitement des fistules digestives à tout niveau chez l'homme.

Exemple n° 8 : application à la culture de cellules endothéliales vasculaires humaines :

A l'état normal, les cellules endothéliales vasculaires (CE) tapissent la surface interne du système cardiovasculaire en s'implantant sur une matrice extracellulaire conjonctive (MEC) sous-endothéliale. Elles forment une monocouche continue inhibée par le contact. La CE est polarisée, sa surface vasculaire n'est pas thrombogène, c'est-à-dire qu'elle ne permet pas physiologiquement l'adhésion de plaquettes, ni l'activation de la coagulation.

Les CE sont isolées à partir de la veine du cordon ombilical par digestion modérée à la collagénase, ensemencées à forte densité sur la surface d'une boîte en polystyrène de 35 mm de diamètre recouverte d'un patch biologique selon l'invention et incubées dans un milieu de culture contenant 30 % de sérum humain, selon une technique précedemment décrite (Biol. Cell. 1984, 52, 9-20). Les cellules prolifèrent et arrivent à confluence en 5 à 6 jours. Les CE confluentes sont examinées en microcospie optique après coloration et en microscopie électronique et présentent les caractères morphologiques des CE. Leur nature endothéliale est confirmée par la présence intracellulaire d'un marqueur spécifique, le facteur de von Willebrand. En outre, les plaquettes humaines n'adhèrent pas à leur surface. Au contraire, les plaquettes adhèrent fortement à la surface thrombogène de l'adduit non recouvert de CE.

Cet adduit se comporte, donc, comme une MEC qui favorise la prolifération d'une monocouche de CE non thrombogènes. Les propriétés de l'adduit en font une MEC de choix pour favoriser la culture de CE humaines et pour le développement de prothèses vasculaires non thrombogènes et biocompatibles.

Exemple n° 9 : étude des propriétés thrombogéniques à l'interface du sang et d'un patch biologique à base d'élastine et de fibrine :

L'interaction du sang avec les surfaces thrombogènes conduit à l'adhésion des plaquettes, la sécrétion du contenu de leurs granules et la formation d'agrégats plaquettaires. L'activation de la coagulation entraîne la génération dethrombine et la polymérisation du fibrinogène en fibrine. Ces réactions qui conduisent dans un vaisseau à la formation d'une thrombose pathologique,-sont souhaitables pour la fixation d'un patch biologique à base d'élastine, de fibrine et éventuellement de collagène utilisé en chirurgie vasculaire, digestive ou cutanée pour la réparation tissulaire.

Le sang humain est collecté sur ACD, les plaquettes sont séparées, lavées, marquées à l'$^{111}$Indium et resuspendues dans le milieu physiologique de Tyrode contenant de l'albumine humaine. La suspension plaquettaire est mise en présence d'un patch de surface connue recouvrant une boite de Petri en polystyrène dans des conditions statiques pendant 30 minutes. L'adhésion des plaquettes à la surface de polystyrène recouverte du patch est comparé à l'adhésion du polystyrène après absorption ou non de diverses protéines.

Le patch à base d'élastine, de fibrine, de fibronectine et de collagène est la surface la plus thrombogène : 42300 ± 1600 plaquettes par mm$^2$ (moyenne ±ESM, n=6). En l'absence d'absorption préalable de protéines, 1600 ± 800 plaquettes par mm$^2$ adhèrent au polystyrène. Le prétraitement de la surface de polystyrène par l'albumine à 0,35 % pendant 30 minutes permet de réduire l'adhésion des plaquettes à 4800 ± 200 par mm$^2$.

Au cours de l'adhésion, les plaquettes sont activées, changent de forme, s'étalent et sécrètent, dans le milieu extracellulaire, le contenu de leurs granules, sérotonine et facteur plaquettaire 4.

Le patch permet l'activation de la voie intrinsèque de la coagulation. L'exposition du plasma humain citraté à la surface du patch entraîne l'activation et la consommation du facteur XII de la phase contact. Ceci se traduit par un allongement du temps de céphaline activé, qui mesure la coagulation globale du plasma.

En conclusion, l'interaction des composants du sang, plaquettes et protéines de la coagulation, à la surface d'un patch de tissu conjonctif artificiel composé d'élastine, de collagène, de fibrine et de fibronectine permet l'activation des plaquettes et de la coagulation. Les propriétés thrombogènes de ce patch favorisent son utilisation comme moyen de réparation des brèches tissulaires.

## Revendications

### Revendications pour les Etats contractants suivants : BE, CH, DE, FR, GB, IT, LI, LU, NL, SE

**1.** Produit à base d'élastine, caractérisé en ce qu'il comprend un adduit tel qu'obtenu par réaction de monomères de fibrine et d'élastine, les monomères de fibrine étant tels qu'engendrés par action de thrombine en présence de $Ca^{++}$ sur du fibrinogène.

**2.** Produit selon la revendication 1, caractérisé en ce que les monomères de fibrine sont tels qu'engendrés par action de thrombine en présence de $Ca^{++}$ sur du plasma.

**3.** Produit selon l'une quelconque des revendications 1 à 2, caractérisé en ce qu'il renferme en outre de la fibronectine.

**4.** Produit selon la revendication 3, caractérisé en ce qu'il est élaboré à partir d'élastine et de cryoglobulines, notamment :
- de cryoglobulines correspondant à la fraction cryodesséchée, dite antihémophilique A, qui comprend environ 10 à 15 g/l de fibrinogène et 2 à 4 g/l environ de fibronectine,
- de cryoglobulines obtenues lors de l'isolement du facteur VIII concentré, qui comprend 9 à 20 g/l environ de fibrinogène et 10 à 14 g/l environ de fibronectine, ou
- de cryoglobulines formées d'un complexe protéique cryodesséché, appelé transglutine, qui comprend de 50 à 90 g/l environ de fibrinogène et de 8 à 15 g/l environ de fibronectine.

**5.** Produit selon l'une quelconque des revendications précédentes, caractérisé en ce qu'il renferme en outre un ou plusieurs additifs destinés notamment à renforcer ses propriétés mécaniques, élastiques et la réticulation du produit recherché, tels que le collagène, la thiourée, des dérivés de cellulose, de l'alginate de calcium, de l'éthylmercrylthiosalicylate de sodium, le complexe FVIII (antihémophilique A).

**6.** Produit selon la revendication 5, caractérisé en ce qu'il se présente sous forme d'une trame réticulée.

**7.** Produit selon l'une quelconque des revendications précédentes, caractérisé en ce que les monomères de fibrine ou les dérivés à partir desquels ils sont produits, et l'élastine sont d'origine humaine.

**8.** Produit selon l'une quelconque des revendications précédentes, caractérisé en ce que les monomères de fibrine, ou les dérivés à partir desquels ils sont produits, et l'élastine sont d'origine animale.

**9.** Procédé de préparation d'un produit à base d'élastine selon la revendication 1, caractérisé en ce qu'il comporte les étapes :
- de mise en contact de fibrinogène ou d'un milieu le renfermant, en particulier de plasma ou des cryoglobulines avec une suspension élastine, en présence d'ions $Ca^{++}$,
- d' addition de thrombine au mélange résultant, suivie d'une étape d'incubation et de séparation de l'adduit formé.

**10.** Procédé selon la revendication 9, caractérisé en ce qu'on effectue l'étape d'incubation à 37 ° C environ, dans un tampon de pH voisin de 7,4 et dont la teneur en $Ca^{++}$ est de l'ordre de 2 mM.

**11.** Procédé selon la revendication 9 ou 10, caractérisé en ce qu'on ajoute successivement à l'élastine les additifs permettant d'améliorer en particulier les propriétés mécaniques, élastiques ainsi que la réticulation du produit recherché, notamment un ou plusieurs additifs tels qu'indiqués dans la revendication 5, puis la fibrine ou un composé ou composition source de fibrine.

**12.** Application des produits selon l'une quelconque des revendications 1 à 8, pour la fabrication de biomatériaux, ou patchs biologiques, en particulier comme tissus conjonctifs artificiels.

**13.** Application des produits selon l'une quelconque des revendications 1 à 8 pour la fabrication de supports pour cultures cellulaires.

**14.** Application des produits selon l'une quelconque des revendications 1 à 8 pour la fabrication de supports pour le greffage de produits biologiques tels que des enzymes ou des inhibiteurs d'enzymes.

**15.** Kit pour culture cellulaire ou réparation tissulaire, caractérisé en ce qu'il comprend un produit selon l'une des revendications 1 à 8, contenu dans un récipient, notamment dans une boîte de pétri, ou dans une enveloppe de conservation et, le cas échéant, un flacon contenant une colle biologique, notamment la Transglutine.

**Revendications pour l'Etat contractant suivant : AT**

**1.** Procédé de préparation d'un produit à base d'élastine comprenant un adduit tel qu'obtenu par réaction de monomères de fibrine et d'élastine, les monomères de fibrine étant tels qu'engendrés par action de thrombine en présence de $Ca^{++}$ sur du fibrinogène, caractérisé en ce qu'il comporte les étapes :
- de mise en contact de fibrinogène ou d'un milieu le renfermant, en particulier de plasma ou des cryoglobulines avec une suspension d'élastine, en présence d'ions $Ca^{++}$.
- d'addition de thrombine au mélange résultant, suivie d'une étape d'incubation et de séparation de l'adduit formé.

**2.** Procédé selon la revendication 1, caractérisé en ce qu'on effectue l'étape d'incubation à 37°C environ, dans un tampon de pH voisin de 7,4 et dont la teneur en $Ca^{++}$ est de l'ordre de 2 mM.

**3.** Procédé selon la revendication 1 ou 2, caractérisé en ce qu'on ajoute successivement à l'élastine les additifs permettant d'améliorer en particulier les propriétés mécaniques, élastiques ainsi que la réticulation du produit recherché, notamment un ou plusieurs additifs tels que le collagène, la thiourée, des dérivés de cellulose, de l'alginate de calcium, de l'éthylmercrythiosalicylate de sodium, le complexe FVIII (antihémophilique A), puis la fibrine ou un composé ou composition source de fibrine.

**4.** Procédé selon l'une quelconque des revendications 1 à 3, caractérisé en ce qu'il conduit à l'obtention d'un produit dans lequel les monomères de fibrine sont tels qu'engendrés par action de thrombine en présence de $Ca^{++}$ sur du plasma.

**5.** Procédé selon l'une quelconque des revendications 1 à 4, caractérisé en ce qu'il renferme en outre de la fibronectine.

**6.** Procédé selon l'une quelconque des revendications précédentes, caractérisé en ce que lorsqu'on met en oeuvre des cryoglobulines, il s'agit notamment :
- de cryoglobulines correspondant à la fraction cryodesséchée, dite antihémophilique A, qui comprend environ 10 à 15 g/l de fibrinogène et 2 à 4 g/l environ de fibronectine,
- de cryoglobulines obtenues lors de l'isolement du facteur VIII concentré, qui comprend 9 à 20 g/l environ de fibrinogène et 10 à 14 g/l environ de fibronectine, ou
- de cryoglobulines formées d'un complexe protéique cryodesséché, appelé transglutine, qui comprend de 50 à 90 g/l environ de fibrinogène et de 8 à 15 g/l environ de fibronectine.

**7.** Procédé selon la revendication 6, caractérisé en ce que le produit obtenu se présente sous forme d'une trame réticulée.

**8.** Procédé selon l'une quelconque des revendications précédentes, caractérisé en ce que les monomères de fibrine ou les dérivés à partir desquels ils sont produits, et l'élastine sont d'origine humaine.

**9.** Procédé selon l'une quelconque des revendications 1 à 7, caractérisé en ce que les monomères de fibrine, ou les dérivés à partir desquels ils sont produits, et l'élastine sont d'origine animale.

**10.** Application des produits tels qu'obtenus selon l'une quelconque des revendications 1 à 9, pour la fabrication de biomatériaux, ou patchs biologiques, en particulier comme tissus conjonctifs artificiels.

**11.** Application des produits tels qu'obtenus selon l'une quelconque des revendications 1 à 9 pour la fabrication de supports pour cultures cellulaires.

**12.** Application des produits tels qu'obtenus selon l'une quelconque des revendications 1 à 9 pour la fabrication de supports pour le greffage de produits biologiques tels que des enzymes ou des inhibiteurs d'enzymes.

**13.** Kit pour culture cellulaire ou réparation tissulaire, caractérisé en ce qu'il comprend un produit tel qu'obtenu selon l'une des revendications 1 à 9, contenu dans un récipient, notamment dans une boîte de pétri, ou dans une enveloppe de conservation et, le cas échéant, un flacon contenant une colle biologique, notamment la Transglutine.

**Claims**
**Claims for the following Contracting States : BE, CH, DE, FR, GB, IT, LI, LU, NL, SE**

**1.** Elastin based product comprising an adduct such as obtained by reacting fibrin monomers and elastin, the fibrin monomers being such as generated by the action of thrombin in the presence of $Ca^{++}$ on fibrinogen.

**2.** A product according to claim 1, wherein the fibrin monomers are such as generated by the action of thrombin in the presence of $Ca^{++}$ on plasma.

**3.** A product according to anyone of claims 1 to 2, further comprising fibronectin.

**4.** A product according to claim 3, characterized in that it is formed from elastin and cryoglobulins, particularly,
- cryoglobulins corresponding to the lyophilized fraction called antihemophilic A, which comprises about 10 to 15 g/l of fibrinogen and about 2 to 4 g/l of fibronectin,
- cryoglobulins obtained during the isolation of concentrated Factor VIII which comprises about 9 to 20 g/l of fibrinogen and about 10 to 14 g/l of fibronectin, and
- cryoglobulins composed of a lyophilized protein complex called Transglutin, which comprises about 50 to 90 g/l of fibrinogen and about 8 to 15 g/l of fibronectin.

**5.** A product according to anyone of the preceding claims, further comprising one or several additives particularly intended for reinforcing the mechanical and elastic properties and cross-linking of the desired product, such as collagen, thiourea, cellulose derivatives, calcium alginate, sodium ethylmercurylthiosalicylate, (antihemophilic A) FVIII complex.

**6.** A product according to claim 5, characterized in that it is under the form of a cross-linked network.

**7.** A product according to anyone of the preceding claims wherein the fibrin monomers or the derivatives from which they are produced, and elastin are of human origin.

**8.** A product according to anyone of the preceding claims wherein the fibrin monomers or the derivatives from which they are produced, and elastin are of animal origin.

**9.** A process for preparing an elastin based product according to claim 1, comprising the steps of :
- contacting fibrinogen or a medium containing the same, particularly plasma or cryoglobulins, with an elastin suspension, in the presence of $Ca^{++}$ ions,
- adding thrombin to the resulting mixture, then
- incubating and separating the formed adduct.

**10.** A process according to claim 9, wherein said incubation step is carried out at about 37° C, in a buffer of a pH of about 7.4, the $Ca^{++}$ content of which is of the order of 2 mM.

**11.** A process according to claim 9 or 10, comprising successively adding to elastin, the additives particularly enabling the mechanical and elastic properties and the cross-linking of the desired product to be improved, particularly one or several additives such as mentioned in claim 5, then fibrin or a compound or composition giving fibrin.

**12.** Use of the products according to anyone of claims 1 to 8, for making biomaterials or biological patchs, particularly as artificial conjonctive tissues.

**13.** Use of the products according to anyone of claims 1 to 8 for making supports for cell cultures.

**14.** Use of the products according to anyone of claims 1 to 8 for making supports for the grafting of biological products such as enzymes or enzyme inhibitors.

**15.** Kits for cell culture or tissue repair, comprising a product according to anyone of claim 1 to 8, contained in a receptacle, particularly a Petri dish, or a storage envelop and, optionally, a flask containing a biological adhesive, particularly Transglutin.

**Claims for the following Contracting State : AT**

**1.** A process for preparing elastin based product comprising an adduct such as obtained by reacting fibrin monomers and elastin, the fibrin monomers being such as generated by the action of thrombin in the presence of $Ca^{++}$ on fibrinogen, comprising the steps of :
- contacting fibrinogen or a medium containing the same, particularly plasma or cryoglobulins, with an elastin suspension, in the presence of $Ca^{++}$ ions,
- adding thrombin to the resulting mixture, then
- incubating and separating the formed adduct.

**2.** A process according to claim 1, wherein said incubation step is carried out at about 37 ° C, in a buffer of a pH of about 7.4, the $Ca^{++}$ content of which is of the order of 2 mM.

**3.** A process according to claim 1 or 2, comprising successively adding to elastin, the additives particularly enabling the mechanical and elastic properties and the cross-linking of the desired product to be improved, particularly one or several additives such as collagen, thiourea, cellulose derivatives, calcium alginate, sodium ethylmercrylthiosalicylate, (antihemophilic A) complex FVIII, then fibrin or a compound or composition giving fibrin.

**4.** A process according to anyone of claims 1 to 3, characterized in that it leads to the obtention of a product wherein the fibrin monomers are such as generated by the action of thrombin in the presence of $C^{++}$ on plasma.

**5.** A process according to anyone of claims 1 to 4 further comprising the use of fibronectin.

**6.** A process according to anyone of the preceding claims, comprising the use of cryoglobulins, particularly,
- cryoglobulins corresponding to the lyophilized fraction called antihemophilic A, which comprises about 10 to 15 g/l of fibrinogen and about 2 to 4 g/l of fibronectin,
- cryoglobulins obtained during the isolation of concentrated Factor VIII which comprises about 9 to 20 g/l of fibrinogen and about 10 to 14 g/l of fibronectin, and
- cryoglobulins composed of a lyophilized protein complex called Transglutin, which comprises about 50 to 90 g/l of fibrinogen and about 8 to 15 g/l of fibronectin.

**7.** A process according to claim 6, characterized in that the obtained product is under the form of a cross-linked network.

**8.** A process according to anyone of the preceding claims, wherein the fibrin monomers, or the derivatives from which they are produced, and elastin are of human origin.

**9.** A process according to anyone of the preceding claims wherein the fibrin monomers, or the derivatives

from which they are produced, and elastin are of animal origin.

**10.** Use of the products such as obtained according to anyone of claims 1 to 9, for making biomaterials or biological patchs, particularly as artificial conjonctive tissues.

**11.** Use of the products such as obtained according to anyone of claims 1 to 9 for making supports for cell cultures.

**12.** Use of the products according to anyone of claims 1 to 9 for making supports for the grafting of biological products such as enzymes or enzyme inhibitors.

**13.** Kits for cell culture or tissue repair, comprising a product such as obtained according to anyone of claims 1 to 9, contained in a receptacle, particularly a Petri dish, or a storage envelop and, optionally, a flask containing a biological adhesive, particularly Transglutin.

**Patentansprüche**
**Patentansprüche für folgende Vertragsstaaten : BE, CH, DE, FR, GB, IT, LI, LU, NL, SE**

**1.** Produkt auf Elastinbasis, dadurch gekennzeichnet, daß es ein Addukt umfaßt, wie es beispielsweise durch Reaktion von Fibrinmonomeren und Elastin erhalten wird, wobei die Fibrinmonomeren solche sind, welche durch Einwirkung von Thrombin in Gegenwart von $Ca^{++}$ auf Fibrinogen entstehen.

**2.** Produkt nach Anspruch 1, dadurch gekennzeichnet, daß die Fibrinmonomeren solche sind, welche durch Einwirkung von Thrombin in Gegenwart von $Ca^{++}$ auf Plasma entstehen.

**3.** Produkt nach Anspruch 1 oder 2, dadurch gekennzeichnet, daß es außerdem Fibronektin unfaßt.

**4.** Produkt nach Anspruch 3, dadurch gekennzeichnet, daß es aus Elastin und Kryoglobulinen hergestellt worden ist, insbesondere aus:
- Kryoglobulinen, welche der gefriergetrockneten, sogenannten antihämophilen Fraktion A entsprechen, welche Fraktion etwa 10 bis 15 g/l Fibrinogen und etwa 2 bis 4 g/l Fibronektin enthalt;
- Kryglobulinen, welche bei der Isolierung des konzentrierten Faktors VIII erhalten worden sind, welcher etwa 9 bis 20 g/l Fibrinogen und etwa 10 bis 14 g/l Fibronektin enthält; oder aus
- Kryoglobulinen, welche aus einem gefriergetrockneten, Transglutin genannten Proteinkomplex gebildet werden, welcher etwa 50 bis 90 g/l Fibrinogen und etwa 8 bis 15 g/1 Fibronektin enthält.

**5.** Produkt nach einem der Ansprüche 1 bis 4, dadurch gekennzeichnet, daß es außerdem einen oder mehrere Zusatzstoffe enthält, welche dazu bestimmt sind, insbesondere die mechanischen und elastischen Eigenschaften, sowie die retikuläre Struktur (Netzwerksstruktur) des gewünschten Produktes, zu verztärken, wie z.B. Kollagen, Thioharnstoff, Zellulosederivate, Kalziumalginat, Natriumethylmerkurithiosalicylat, und den Komplex FVIII (antihämophiles Globulin A).

**6.** Produkt nach Anspruch 5, dadurch gekennzeichnet, daß es die Form einer retikulären Folie (Netzwerksfolie) hat.

**7.** Produkt nach einem der Ansprüche 1 bis 6, dadurch gekennzeichnet, daß die Fibrinmonomeren oder die Derivate, aus denen sie erzeugt worden sind, ebenso wie das Elastin menschlichen Ursprungs sind.

**8.** Produkt nach einem der Ansprüche 1 bis 7, dadurch gekennzeichnet, daß die Fibrinmonomeren oder die Derivate, aus denen sie erzeugt worden sind, ebenso wie das Elastin, tierischen Ursprungs sind.

**9.** Verfahren zur Herstellung eines Produktes auf Elastinbasis nach Anspruch 1, dadurch gekennzeichnet, daß dieses Verfahren die Stufen:
- des Zusammenbringens von Fibrinogen oder eines dieses enthaltenden Mediums, insbesondere von Plasma oder von Kryoglobulinen, mit einer Elastinsuspension in Gegenwart von $Ca^{++}$-Ionen;
- der Zugabe von Thrombin zu dem so entstandenen Gemisch, mit einer anschließenden Stufe der Inkubation und der Abtrennung des gebildeten Adduktes;

umfaßt.

**10.** Verfahren nach Anspruch 9, dadurch gekennzeichnet, daß man die Inkubationsstufe bei etwa 37° C in einem Puffer mit einem in der Nahe von 7,4 liegenden pH-Wert, wobei der Gehalt des Puffers an $Ca^{++}$ in der Größenordnung von 2 mM liegt, durchführt.

**11.** Verfahren nach Anspruch 9 oder 10, dadurch gekennzeichnet, daß man zu dem Elastin nacheinander die Zusatzstoffe hinzufügt, welche es ermöglichen, insbesondere die mechanischen und elastischen Eigenschaften, ebenso wie die retikuläre Struktur (Netzwerksstruktur) des gewünschten Produktes zu verbessern, insbesondere einen oder mehrere Zusatzstoffe, wie dieselben im Anspruch 5 angeführt worden sind, und dann Fibrin oder eine als Quelle für Fibrin dienende Verbindung oder Zusammensetzung hinzufügt.

**12.** Verwendung der Produkte nach einem der Ansprüche 1 bis 8 zur Erzeugung von Biomaterialien, oder von biologischen "Patch-graft"-Materialien, insbesondere als künstliches Bindegewebe.

**13.** Verwendung der Produkte nach einem der Ansprüche 1 bis 8 für die Erzeugung von Trägern für Zellkulturen.

**14.** Verwendung der Produkte nach einem der Ansprüche 1 bis 8 für die Erzeugung von Trägern zum Übertragen von biologischen Produkten, wie z.B. Enzymen oder Enzyminhibitoren.

**15.** Kit zur eine Zellkultur oder eine Gewebewiederherstellung, dadurch gekennzeichnet, daß er ein Produkt nach einem der Ansprüche 1 bis 8, welches in einem Behälter, insbesondere in einer Petrischale oder in einer konservierenden Umhüllung enthalten ist, und gegebenenfalls ein Fläschchen unfaßt, welches einen biologischen Klebstoff, insbesondere Transglutin, enthält.

**Patentansprüche für folgenden Vertragsstaat : AT**

**1.** Verfahren zur Herstellung eines Produktes auf Elastinbasis, welches ein Addukt umfaßt, wie es beispielsweise durch Reaktion von Fibrinmonomeren und Elastin erhalten wird, wobei die Fibrinmonomeren solche sind, welche durch Einwirkung von Thrombin in Gegenwart von $Ca^{++}$ auf Fibrinogen entstehen, dadurch gekennzeichnet, daß dieses Verfahren die Stufen:
- des Zusammenbringens von Fibrinogen oder eines dieses enthaltenden Mediums, insbesondere von Plasma oder von Kryoglobulinen, mit einer Elastinsuspension in Gegenwart von $Ca^{++}$-Ionen;
- der Zugabe von Thrombin zu dem so entstandenen Gemisch, mit einer anschließenden Stufe der Inkubation und der Abtrennung des gebildeten Adduktes;

umfaßt.

**2.** Verfahren nach Anspruch 1, dadurch gekennzeichnet, daß man die Inkubationsstufe bei etwa 37° C in einem Puffer mit einem in der Nahe von 7,4 liegenden pH-Wert, wobei der Gehalt des Puffers an $Ca^{++}$ in der Größenordnung von 2 mM liegt, durchführt.

**3.** Verfahren nach Anspruch 1 oder 2, dadurch gekennzeichnet, daß man zu dem Elastin Zusatsstoffe hinzufügt, welche es ermöglichen, insbesondere die mechanischen und elastischen Eigenschaften, wie z.B. die retikuläre Struktur (Netzwerksstruktur) des gewünschten Produktes, zu verbessern, insbesondere einen oder mehrere Zusatzstoffe, wie z. B. Kollagen, Thioharnstoff, Zellulosederivate, Kalziumalginat, Natriumethylmerkurithiosalicylat, den Komplex FVIII (antihämophiles Globulin A), dann Fibrin oder eine als Quelle für Fibrin dienende Verbindung oder Zussammensetzung.

**4.** Verfahren nach einem der Ansprüche 1 bis 3, dadurch gekennzeichnet, daß es zur Erzielung eines Produktes führt, in welchem die Fibrinmonomeren solche sind, welche durch die Einwirkung von Thrombin auf Plasma, in Gegenwart von $Ca^{++}$, entstanden sind.

**5.** Verfahren nach einem der Ansprüche 1 bis 4, dadurch gekennzeichnet, daß es außerdem das Fibronektin umfaßt.

**6.** Verfahren nach einem der Ansprüche 1 bis 5, dadurch gekennzeichnet, daß es sich dann, wenn man Kryoglobuline einsetzt, insbesondere handelt um:
- Kryoglobuline, welche der gefriergetrockneten, sogenannten antihämophilen Fraktion A entsprechen, welche Fraktion etwa 10 bis 15 g/l Fibrinogen und etwa 2 bis 4 g/l Fibronektin enthält;
- Kryoglobuline, welche bei der Isolierung des konzentrierten Faktors VIII erhalten worden sind, welcher etwa 9 bis 20 g/l Fibrinogen und etwa 10 bis 14 g/l Fibronektin enthält; oder um
- Kryoglobuline handelt, welche aus einem gefriergetrockneten, Transglutin genannten Proteinkomplex gebildet werden, welcher etwa 50 bis 90 g/l Fibrinogen und etwa 8 bis 15 g/l Fibronektin enthält.

**7.** Verfahren nach Anspruch 6, dadurch gekennzeichnet, daß das erhaltene Produkt die Form einer retikulären Folie (Netzwerksfolie) hat.

**8.** Verfahren nach einem der Ansprüche 1 bis 7, dadurch gekennzeichnet, daß die Fibrinmonomeren oder die Derivate, aus denen sie erzeugt worden sind, ebenso wie das Elastin menschlichen Ursprungs sind.

**9.** Verfahren nach einem der Ansprüche 1 bis 7, dadurch gekennzeichnet, daß die Fibrinmonomeren oder die Derivate, aus denen sie erzeugt worden sind, ebenso wie das Elastin, tierischen Ursprungs sind.

**10.** Verwendung der Produkte wie sie nach einem der Ansprüche 1 bis 9 erhalten worden sind, zur Erzeugung von Biomaterialien, oder von biologischen "Patch-graft"-Materialien, insbesondere als künstliches Bindegewebe.

**11.** Verwendung der Produkte, wie dieselben nach einem der Ansprüche 1 bis 9 erhalten worden sind, für die Erzeugung von Trägern für Zellkulturen.

**12.** Verwendung der Produkte, wie sie nach einem der Ansprüche 1 bis 9 erhalten worden sind, für die Erzeugung von Trägern zum Übertragen von biologischen Produkten, wie z.B. Enzymen oder Enzyminhibitoren.

**13.** Kit für eine Zellkultur oder eine Gewebewiederherstellung, dadurch gekennzeichnet, daß er ein Produkt, wie es nach einem der Ansprüche 1 bis 9 erhalten worden ist und welches in einem Behälter, insbesondere in einer Petrischale oder in einer konservierenden Umhüllung enthalten ist, und gegebenenfalls ein Fläschchen umfaßt, welches einen biologischen Klebstoff, insbesondere Transglutin, enthält.

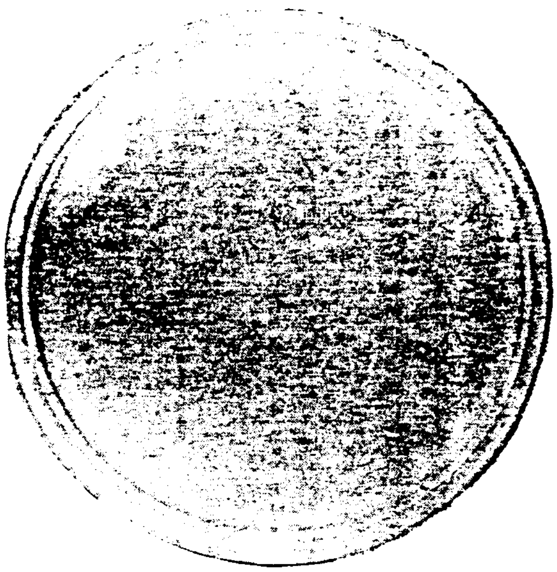

FIG.1a.

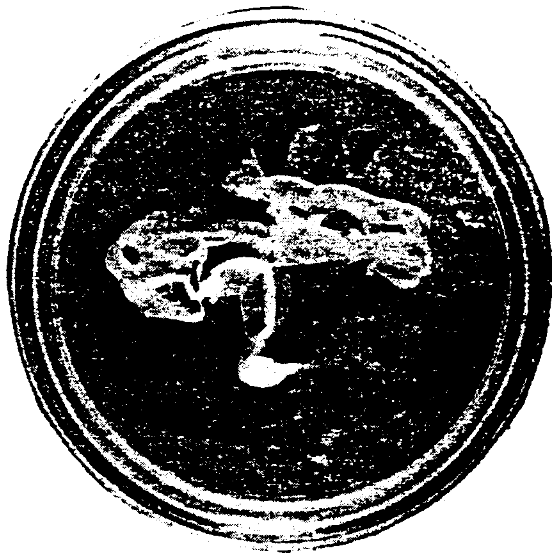

FIG.1b.

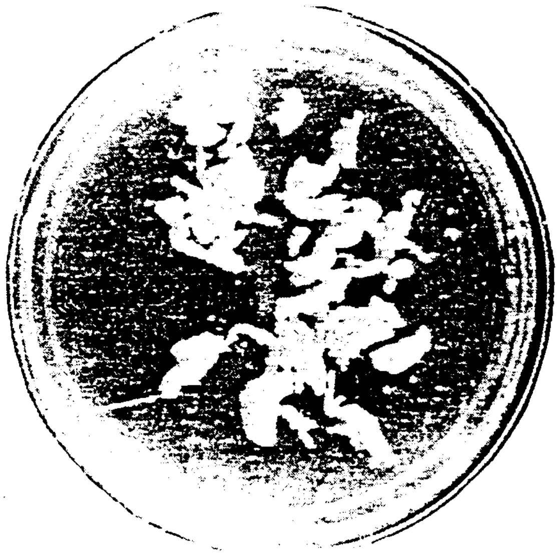

FIG.1c.

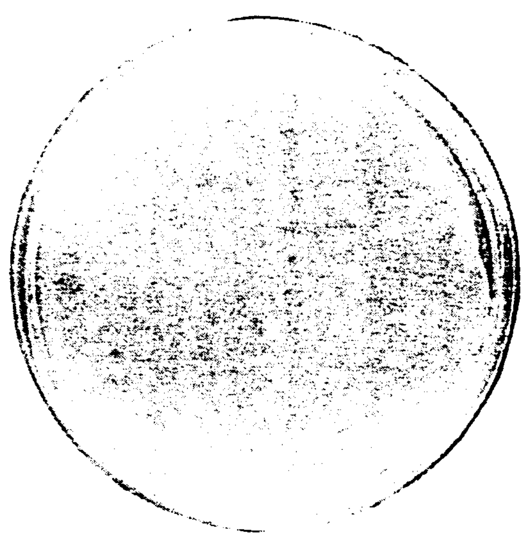

FIG.1d.

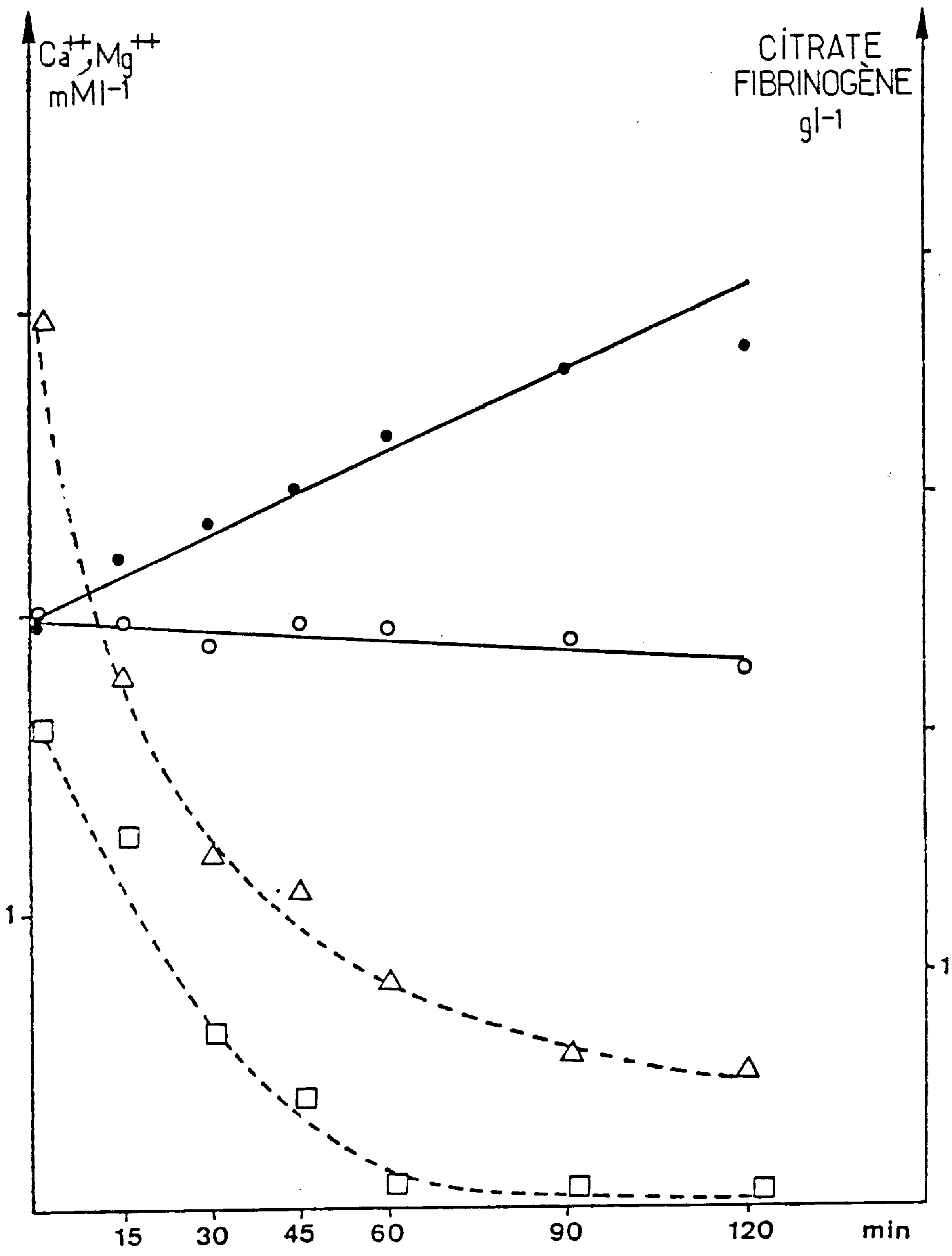


FIG.2.

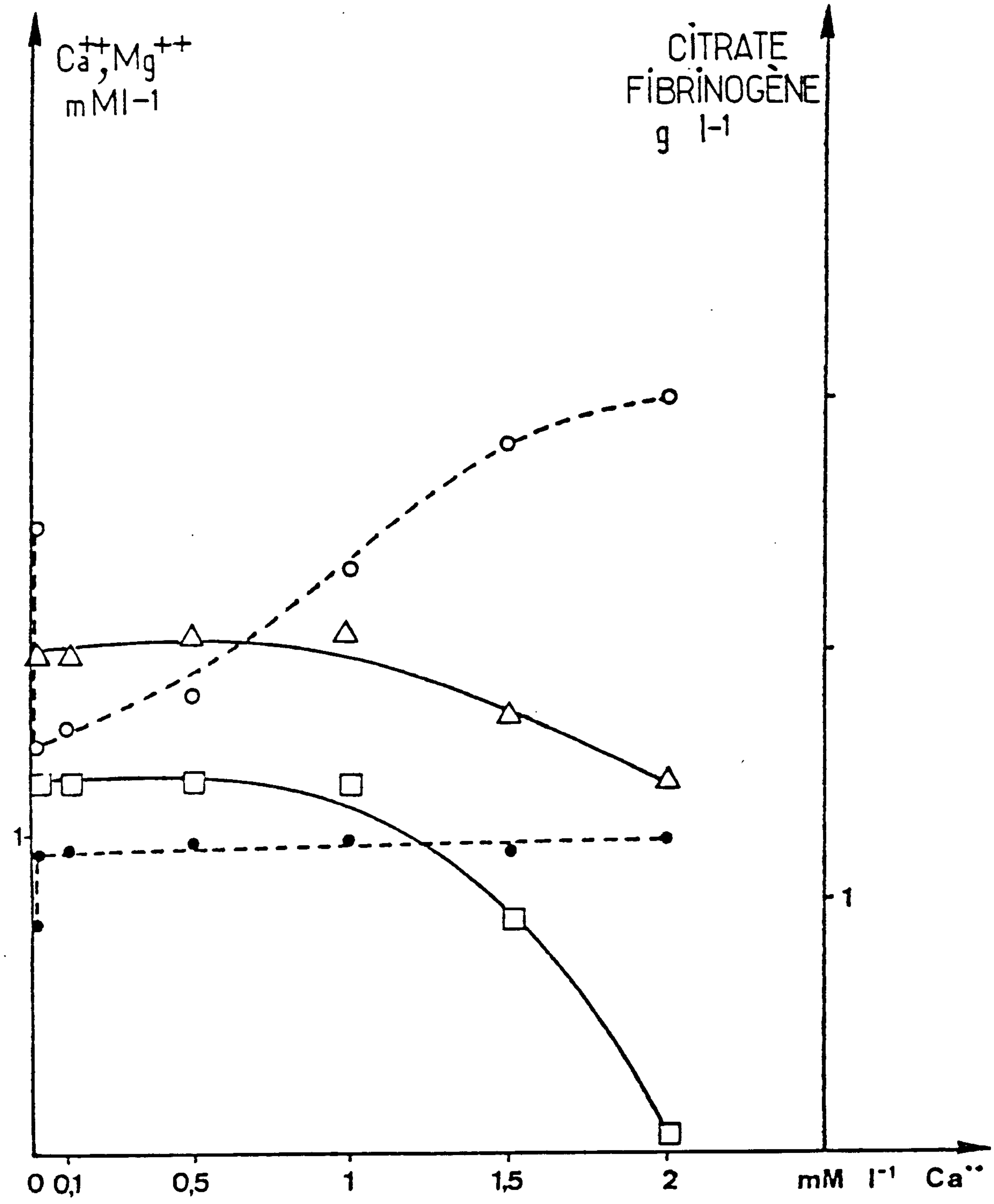


FIG.3.

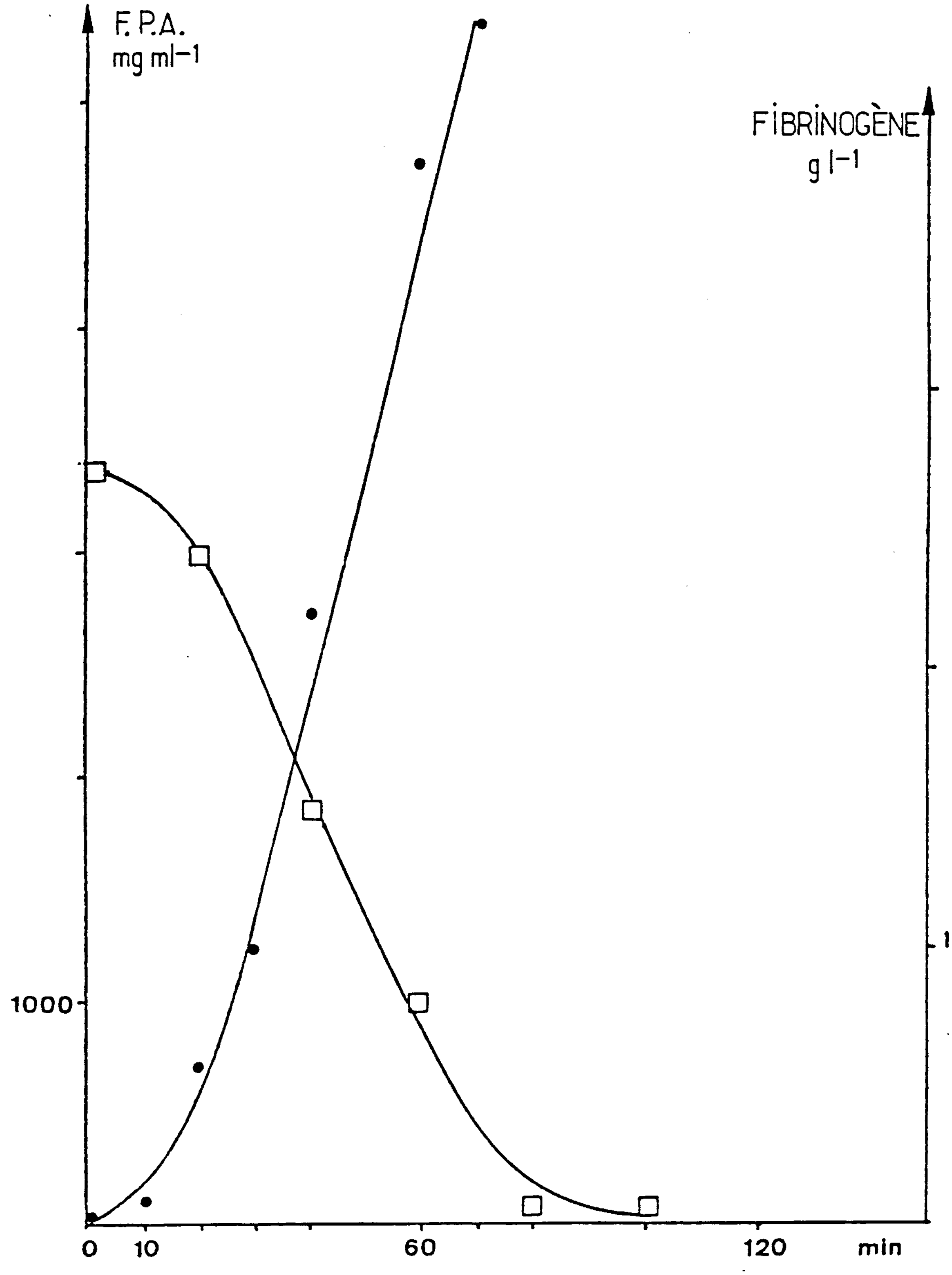


FIG.4.

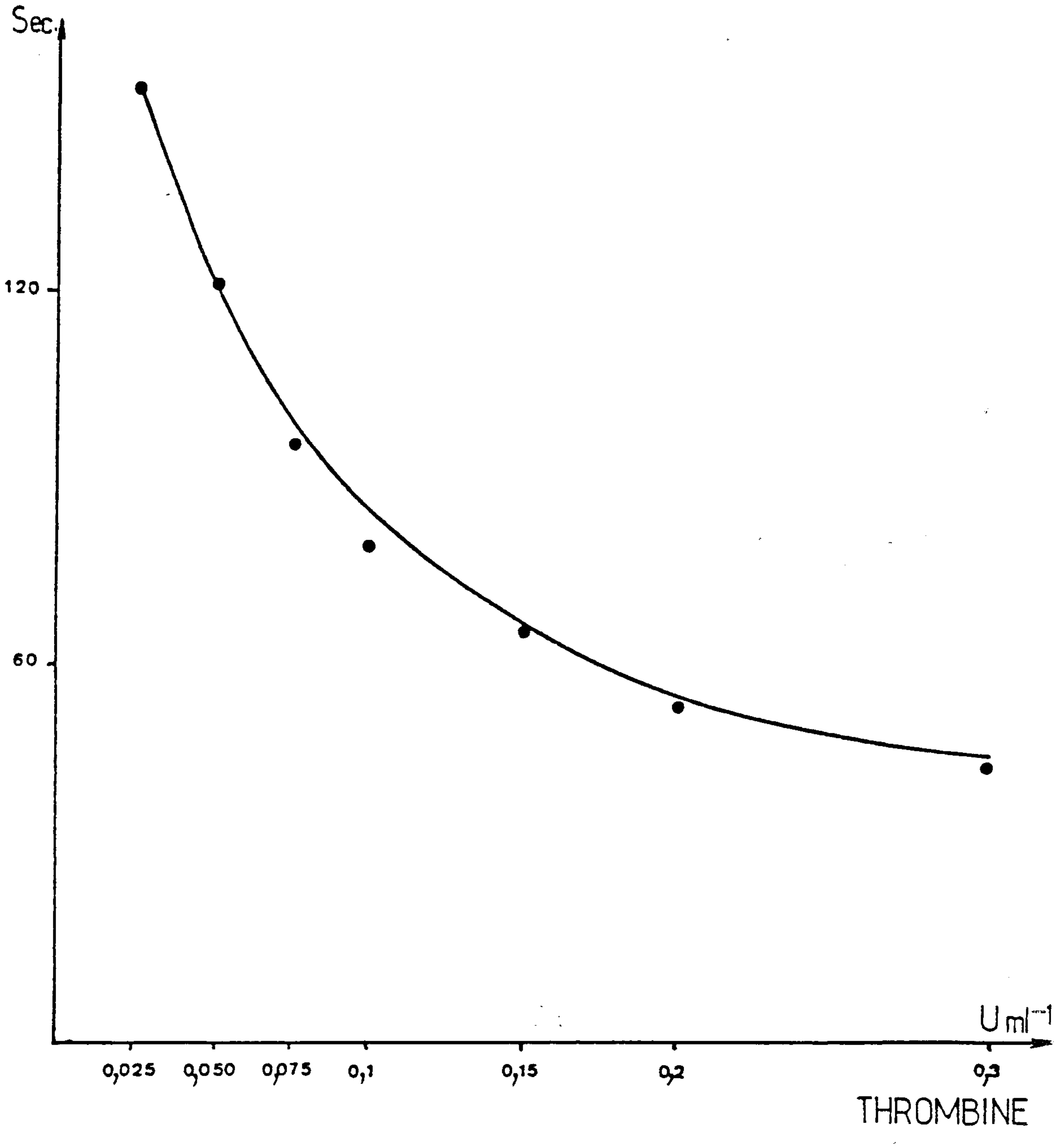


FIG.5.